# EUROPEAN PATENT APPLICATION

(11) **EP 4 488 678 A1**
(43) Date of publication of application: **08.01.2025**
(21) Application number: 23760212.3
(22) Date of filing: 28.02.2023
(51) Int. Cl.: G01N 33/00, C07K 14/705, C12M 1/00, G01N 33/02, G06Q 50/10

(54) **INFORMATION PROCESSING DEVICE, CARTRIDGE, RELEASE DEVICE, N-DIMENSIONAL CODE, CONTROL METHOD, AND COMPUTER PROGRAM**

(30) Priority: 28.02.2022 WO PCT/JP2022/008416; 12.04.2022 JP 2022065877
(71) Applicant: Komi Hakko Corporation, Osaka-shi, Osaka 550-0002 (JP)
(72) Inventor: KUBO Kenji, Osaka-shi, Osaka 550-0002 (JP); ASAI Naoto, Suita-shi, Osaka 565-0871 (JP)
(74) Representative: Meissner Bolte Partnerschaft mbB
(86) International application number: PCT/JP2023/007331
(87) International publication number: WO 2023/163214

(57) **Abstract**

An information processing device comprising: an acquisition means for acquiring receptor response information indicating responses of a plurality of types of olfactory receptors or taste receptors; a storage means for storing the receptor response information; an emission information determination means for determining emission information related to emission of a plurality of types of substances related to reception of scent or taste according to the receptor response information; and an emission control means for causing emission of at least one substance of the plurality of types of substances related to reception of scent or taste based on the emission information.

## Description

### TECHNICAL FIELD

The present invention relates to an information processing device, a cartridge, an emission device, an N-dimensional code, a control method, and a computer program.

### BACKGROUND ART

In recent years, technologies related to methods for quantifying scents have been proposed. For example, Patent Document 1 discloses a technique for measuring the degree of activation of individual receptors by applying a predetermined ligand to a receptor array in which predetermined receptors are comprehensively arranged. Patent Document 1 also discloses a method for reproducing the scent of a target substance by combining two or more standard substances (fragrances) based on the measured activation level of each receptor of the target substance and the standard substance.

### Citation List

### Patent Document

Patent Document 1: PCT International Publication No. WO2019/035476

### DISCLOSURE OF THE INVENTION

### Problems to be Solved by the Invention

However, while multiple scent- or taste-receptive substances are combined to reproduce a target scent, there is a great number of combination types and combination methods of such substances, and thus it is difficult to reproduce the target scent.

It is an object of the present invention to reproduce a target scent or taste without relying on a personal skill. Means for Solving the Problems

In order to achieve the above object, one aspect of the present invention is an information processing device including: an acquisition unit configured to acquire receptor response information indicating responses of a plurality of types of olfactory receptors or taste receptors; a storage unit configured to store the receptor response information; an emission information determination unit configured to determine emission information related to emission of a plurality of types of substances related to reception of scent or taste according to the receptor response information; and an emission control unit configured to cause emission of at least one substance of the plurality of types of substances related to reception of scent or taste based on the emission information.

In order to achieve the above object, one aspect of the present invention is a cartridge to be filled with a substance related to reception of the scent or taste emitted by the information processing device described above, the cartridge being filled with a composition that makes only a specific receptor selectively respond.

In order to achieve the above object, one aspect of the present invention is an emission device including: a mounting section for mounting the cartridge described above; and an emission port from which the substance related to reception of the scent or taste is emitted based on emission control performed by the emission control unit.

In order to achieve the above object, one aspect of the present invention is a creation method for scent or taste, the method including a step of emitting a substance related to reception of the scent or taste by the emission device described above.

In order to achieve the above object, one aspect of the present invention is a data structure containing the receptor response information used in the information processing device described above.

In order to achieve the above object, one aspect of the present invention is a control method including: an encoding step of converting the receptor response information into an N-dimensional code by performing predetermined encoding on the receptor response information.

In order to achieve the above object, one aspect of the present invention is a control method for an information processing device, the method including: an acquisition step of acquiring receptor response information indicating responses of a plurality of types of olfactory receptors or taste receptors; an emission information determination step of determining emission information related to emission of substances related to reception of a plurality of types of scents or tastes according to the receptor response information; and an emission control step of causing emission of at least one substance of the plurality of types of substances related to reception of scent or taste based on the emission information.

In order to achieve the above object, one aspect of the present invention is a computer program to cause a computer to execute: an acquisition step of acquiring receptor response information indicating responses of a plurality of types of olfactory receptors or taste receptors; an emission information determination step of determining emission information related to emission of substances related to reception of a plurality of types of scents or tastes according to the receptor response information; and an emission control step of causing emission of at least one substance of the plurality of types of substances related to reception of scent or taste based on the emission information.

### Effects of the Invention

According to the present invention, a target scent or taste can be reproduced in a non-attributable manner.

### BRIEF DESCRIPTION OF THE DRAWINGS

FIG. 1 illustrates an overview of a scent reproduction method according to an embodiment;
FIG. 2 illustrates an overview of the scent reproduction method according to the embodiment;
FIG. 3 is a schematic configuration diagram of a spray control system;
FIG. 4 is a block diagram illustrating a hardware configuration of a server;
FIG. 5 is a functional block diagram illustrating an example of a functional configuration of the server;
FIG. 6 is a flowchart illustrating operation of a spray control process;
FIG. 7 illustrates an overview of a scent reproduction method according to another embodiment;
FIG. 8 is a block diagram illustrating a configuration of a conversion device;
FIG. 9 is an example of a graph showing correlation between values of target variables when converted using the conversion device and values after conversion;
FIG. 10 is an example of a graph showing correlation between values of object variables when converted using the conversion device and values after conversion;
FIG. 11 is an example of a graph showing correlation between values of object variables when converted using the conversion device and values after conversion;
FIG. 12 is an example of a graph showing correlation between values of object variables when converted using the conversion device and values after conversion;
FIG. 13 is a block diagram illustrating a configuration of a prediction model generation device;
FIG. 14 is an example of a graph showing correlation between values of object variables when converted using the conversion device and values after conversion; and
FIG. 15 is an example of a graph showing correlation between values of object variables when converted using the conversion device and values after conversion.

### PREFERRED MODE FOR CARRYING OUT THE INVENTION

### (First Embodiment)

### <Overview>

In general, when receptors are olfactory receptors, scent molecules bind to proteins called olfactory receptors that are expressed on the surface of olfactory nerve cells in the nasal cavity. This triggers the transmission of information within the olfactory nerve cells and the transmission of scent information to the brain. Based on genome analysis, it is believed that there are approximately 400 types of olfactory receptors in humans. Further, it is understood that certain groups of olfactory receptors are activated to different degrees in response to specific scent molecules, and that these stimuli are transmitted to the brain and combined to identify various scents in the brain.

This embodiment will deal with an example in which a scent is reproduced based on the results of measuring the activity of individual receptors by applying a predetermined ligand to a receptor array in which olfactory receptors (ORs) are comprehensively arranged, as described above. In this embodiment, an example will be described in which a plurality of receptors (e.g., 30 different receptors) that largely influence human perception are used as the predetermined olfactory receptors. Note that this test can be performed not only for olfactory receptors but also for taste receptors. Now, the embodiment will be described with reference to the drawings.

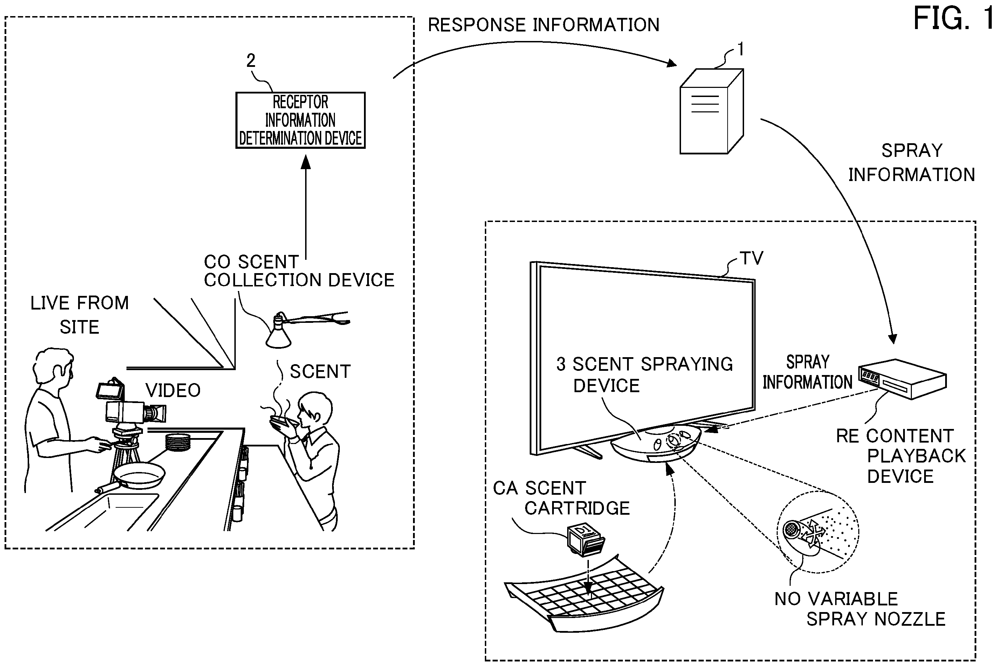

FIG. 1 illustrates an overview of a scent reproduction method to which an information processing device according to the present embodiment is applied. FIG. 1 illustrates an example in which content advertising food or a beverage (e.g., ramen) at a restaurant is distributed to a viewer, and the smell of the food or beverage is reproduced on the viewer's side by using a predetermined device. Note that examples of the content include content distributed via terrestrial broadcasting, satellite broadcasting, the Internet, and the like.

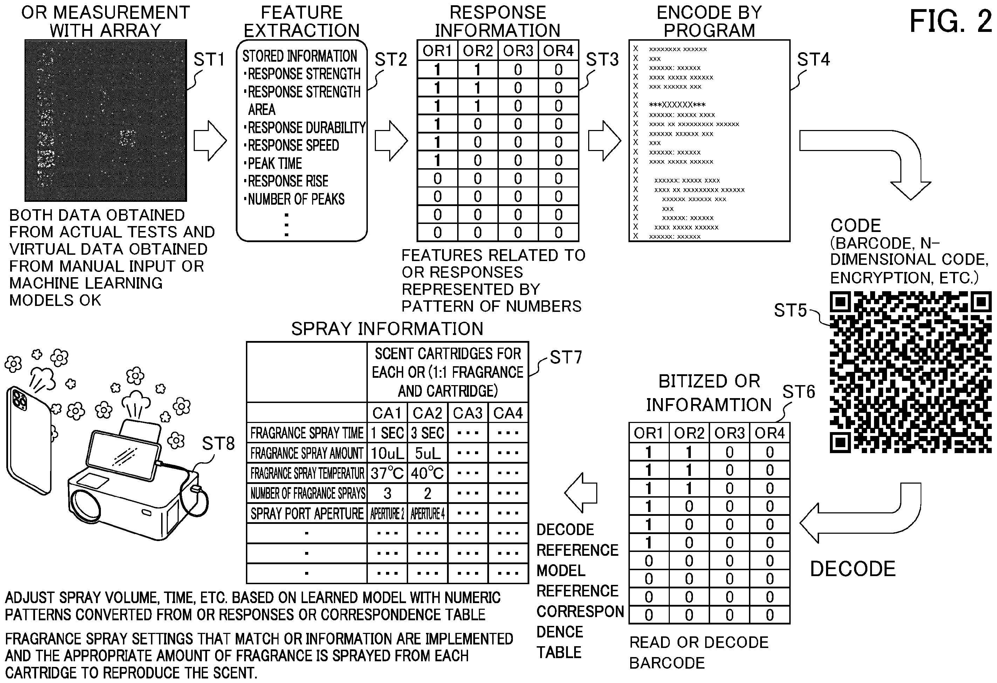

In the example illustrated in FIG. 1, a predetermined camera captures images of visitors consuming food or a beverage at a restaurant, and a scent collection device CO collects the scent (e.g., gas) of the food or beverage. The collected scent is analyzed by a receptor information determination device 2 and converted into response information for a predetermined receptor. The converted response information is sent to a server 1. The server 1 then sends the response information along with the content to individual content playback devices RE. The content playback device RE sends the above-described content to a predetermined display device TV based on a request to view the content. The content playback device RE also transmits, to a scent spraying device 3, spray information based on the response information corresponding to the above-described content. The scent spraying device 3 includes multiple types of scent cartridges CA, and fragrances (e.g., gas, liquid or powder) extracted from the multiple types of scent cartridges CA are sprayed (ejected, jetted) via variable spray nozzles (nozzles NO). The response information and spray information are explained with reference to FIG. 2. In the present embodiment, an example in which a fragrance is emitted from an emission device (scent spraying device 3) will be described. However, the object to be emitted is not limited to a fragrance and can be any substance related to scent or taste reception. Substances related to scent or taste reception include agonists, antagonists, modulators, and the like. An agonist is a substance that augments or expresses an action when it binds to a receptor. An antagonist is a substance that acts in an inhibitory manner when it binds to a receptor or prevents the binding of other agonists. A modulator is a substance that is not itself stimulus-responsive but amplifies or decreases an agonist's or antagonist's stimulus response. In other words, a modulator is a substance that binds to a site different from the ligand bond site to increase or decrease the action of an olfactory receptor or taste receptor.

FIG. 2 is a diagram illustrating an example of the flow of extracting features from a collected scent, generating response information, and finally generating spray information.

At code ST1, the receptor information determination device 2 applies a predetermined ligand (scent molecule) to a receptor array made up of comprehensively arranged predetermined receptors, and measures the activity level of each receptor (primary receptor response information). The method of measuring the activity level of each receptor is not particularly limited and may be, for example, acquiring data by actual testing, as described in Patent Document 1 or described later, or collecting manually entered data or virtual data obtained from a machine learning model.

At code ST2, the receptor information determination device 2 extracts features of a target scent on the basis of the measured activity of each receptor. The method of extracting features is not particularly limited and, for example, the method described in Patent Document 1 can be used.

The features are response characteristics of olfactory receptors to a test substance (response information; receptor response information), such as response intensity of each receptor, area of response intensity, response durability, response speed, peak time, rise (timing) of response, or number of peaks.

At code ST3, the response information (secondary receptor response information) of each receptor is generated based on the features described above. In the example in FIG. 2, four types of receptors OR1 to OR4 are illustrated, but the type (quantity) of receptors is not limited. In the example in FIG. 2, the feature of each receptor is expressed as a binary number, but the method of quantifying the feature is not limited. For example, the feature may be expressed as other decimal numbers, such as decimal, or as a string of characters. In other words, the response information is features of scent molecules at multiple types of receptors. For example, the response information is information indicating at least one feature of response intensity, area of response intensity, response durability, response speed, peak time, rise of response, and/or number of peaks. Note that the response information is not limited to the above and may be various parameters for scent molecules at the multiple types of receptors.

At codes ST4 and ST5, the response information described above is encoded and converted into an N-dimensional code (where N is an integer greater than or equal to 1). Then, the N-dimensional code is transmitted. In the present embodiment, the N-dimensional code is described as a two-dimensional code. The two-dimensional code according to this embodiment has a data part in which a bit sequence corresponding to target data is represented as a pattern of white or black cells representing constituent units of a symbol arranged in a matrix, and one or more finder patterns that are arranged separately from the data part. The data part described above includes information based on receptor response information (e.g., the response characteristics and response information described above) obtained using olfactory receptors or taste receptors that are responsive to extracellular substances (e.g., scent molecules). In other words, the code ST4 is data used to execute a control method for an information processing device (server 1) to determine emission information regarding the release of a substance related to the reception of a plurality of types of odors or tastes, and can also be regarding as an encoding step of converting the (receptor) response information into the N-dimensional code (e.g., two-dimensional code) by performing predetermined encoding on the data corresponding to the (receptor) response information.

At code ST6, the N-dimensional code described above is decoded at the viewer's side and converted to the original response information. In other words, code ST6 is a control method for the information processing device (server 1) and can also be regarded as a decoding step of converting the N-dimensional code into the (receptor) response information by performing predetermined decoding on the N-dimensional code (e.g., two-dimensional codes).

At code ST7, for example, the response information is converted by the server 1 described above into spray information used for spraying the reproduced scent. FIG. 2 illustrates the spray information for the cartridges CA1 to CA4 corresponding to the receptors OR1 to OR4. In other words, the cartridge CA1 is filled with a fragrance to stimulate the receptor OR1. Although FIG. 2 illustrates an example of a one-to-one correspondence between receptor and cartridge, no limitation is intended and more types of cartridges than the types (number) of receptors may be used, or fewer types of cartridges may be used. That is, one cartridge CA may be able to give a response (agonize) or adjust a response (antagonize or modulate) at multiple receptors. Using a cartridge CA that stimulates multiple receptors makes it possible to reduce the size of the scent sprayer 3 and lower costs. For example, when stimulating the receptors OR1 and OR3, the following cartridge CA may be used to spray the scent.
- Cartridge CA filled with substance that stimulates the receptors OR1 and OR3

For example, the following three types of cartridges CA may be used to spray the scent when stimulating the receptors OR1 to OR5.
- Cartridge CA filled with substance (agonist) that stimulates the receptors OR1, OR3, OR5, OR6
- Cartridge CA filled with substance (agonist) that stimulates the receptors OR2, OR4, OR6
- Cartridge CA filled with substance (antagonist) that prevents the binding of other substances to the receptor OR6.

The spray information is information for reproducing a target scent by spraying one or a combination of two or more of several types of fragrances. Examples of the information include spray time, spray amount, spray temperature, number of fragrance sprays, spray port aperture, and spray direction of the fragrance.

At code ST8, based on the spray information described above, the scent spraying device 3 sprays a fragrance to provide a scent (fragrance) that reproduces the target scent to the content viewer. Various devices such as display devices (TVs), smart phones, projection devices (projectors), and HMDs (head-mounted displays) can are examples of the scent spraying device 3. Note that the scent does not have to be related to the content. For example, a predetermined user-specified scent may be sprayed. In this case, the scent item and the response information or spray information corresponding to the scent item is held in a predetermined storage unit in advance.

In the above example, the N-dimensional code in which the target scent is replaced with electronic data is used as digital scent information. However, for example, the extracted feature (code ST2) or secondary receptor response information (code ST3) described above may be used (or distributed) as the digital scent information. In other words, the feature, the secondary receptor response information, or the N-dimensional code described above can also each be regarded as indicating the data structure of the receptor response information used in the server 1 (information processor). Note that, in the case of reproducing the target scent, as in the present embodiment, a substance related to scent reception (e.g., a fragrance) is sprayed (emitted). However, in the case of reproducing a target taste, a substance related to taste reception (e.g., a seasoning) is emitted, as described below as a variant. In other words, "spraying" as used in the present embodiment is a sub-concept of "emission".

### <System Configuration>

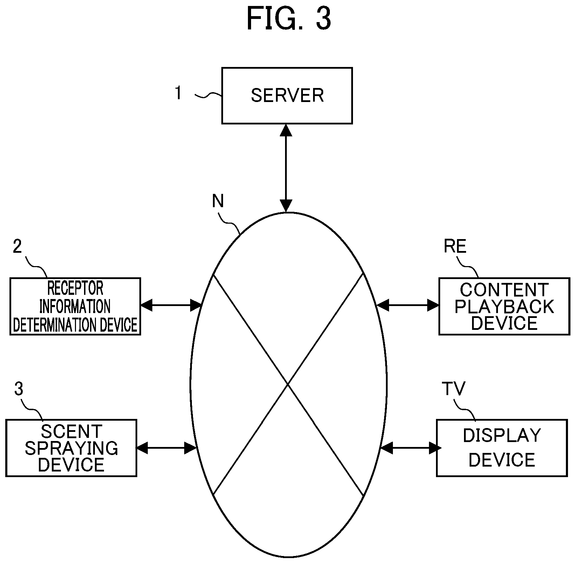

FIG. 3 illustrates an overview of a system configuration of a spray control system (scent reproduction system) according to the present embodiment. The spray control system according to the present embodiment includes the server 1, a collection device CO, the receptor information determination device 2, a content playback device RE, a display device TV, and a scent spraying device 3, which are connected to each other via a predetermined network N such as the Internet. The server 1 executes various processes in cooperation with the operation of each of the receptor information determination device 2, the content playback device RE, the display device TV, and the scent spraying device 3.

The scent collection device CO collects a scent corresponding to the content. As illustrated in FIG. 1, the scent may be collected in real time when capturing video related to the content, or at a different time than when capturing video related to the content.

The receptor information determination device 2 analyzes the scent collected by the scent collection device CO described above. The receptor information determination device 2 may analyze the components contained in the scent (e.g., gases and liquids). However, in the present embodiment, receptor response information obtained using olfactory receptors that are responsive to the scent (scent molecules) described above (the result of applying a predetermined ligand to a receptor array made up of receptors arranged comprehensively and measuring the activity of each receptor) is analyzed. With this configuration, when reproducing the scent, the target scent can be reproduced using a component different from the target scent. Note that the receptor information determination device 2 may analyze response characteristics using receptors as described in Patent Document 1 or may analyze response characteristics from components contained in the scent molecules.

The content playback device RE displays (plays) video content or the like on the display device TV. The content playback device RE also sends the spray information to the scent spraying device 3 such that the fragrance is sprayed from the scent spraying device 3 at a predetermined timing of the related video content. Note that, in the present embodiment, a display device TV such as digital signage can also be used. In this configuration, the content playback device RE is provided, but the content may be sent directly from the server 1 to the display device TV, or the spray information may be sent directly from the server 1 to the scent spraying device 3.

The display device TV displays the content. In this embodiment, an example in which the display device TV is provided with the scent spraying device 3 (described below) will be described, but the display device TV and the scent spraying device 3 may be independent and separate devices. The display device TV will be described as a display device as an example, but no limitation is intended and the display device TV may be a projection device such as a projector, for example.

The scent spraying device 3 reproduces the target scent by spraying a fragrance based on the spray information. In the present embodiment, the scent spraying device 3 is described as a mounting section (not shown) for mounting the cartridge CA (scent cartridge) and the nozzle NO (variable spray nozzle) as an example, but no limitation is intended. The cartridge CA is a cartridge to be filled with a fragrance to be sprayed by a predetermined information processing device (the server 1, the content playback device RE, etc.) and is filled with a composition that makes only a specific receptor selectively respond. Note that the composition is not limited to a composition that makes only a specific receptor selectively respond and may be a composition that makes multiple receptors respond. Multiple types of the cartridges CA having different compositions are mounted in the scent spraying device 3. The nozzle NO is a spray port from which a predetermined fragrance is sprayed based on spray control (spray information) by a predetermined information processing device (the server 1, the content playback device RE, etc.).

### <Hardware Configuration>

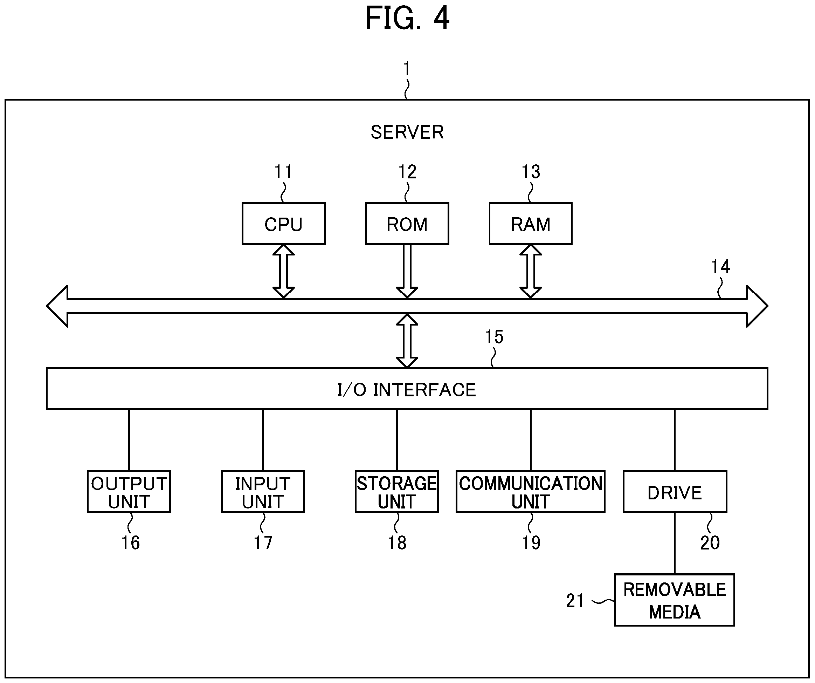

FIG. 4 is a block diagram illustrating the hardware configuration of the server 1 according to the present embodiment. The server 1 includes a central processing unit (CPU) 11, a read-only memory (ROM) 12, a random-access memory (RAM) 13, a bus 14, an input/output (I/O) interface 15, an output unit 16, an input unit 17, a storage unit 18, a communication unit 19, and a drive 20.

The CPU 11 executes various processes according to programs recorded in the ROM 12 or loaded into the RAM 13 from the storage unit 18. The R_AM 13 also stores data and other information necessary for the CPU 11 to perform various processes. The CPU 11, the ROM 12, and the RAM 13 are connected to each other via the bus 14. The I/O interface 15 is also connected to the bus 14.

The output unit 16, the input unit 17, the storage unit 18, the communication unit 19, and the drive 20 are connected to the I/O interface 15. The output unit 16 consists of a display or a speaker, for example, and outputs various types of information as images or sound. The input unit 17 consists of a keyboard or a mouse to input various information. The memory section 18 consists of a hard disk, a dynamic random access memory (DRAM), or the like and stores various data. The communication unit 19 communicates with other devices via a network N, including the Internet.

The removable media 21 consisting of a magnetic disk, an optical disk, a magneto-optical disk, a semiconductor memory, or the like is mounted on the drive 20 as appropriate. A program read by the drive 20 from the removable media 21 is installed in the storage unit 18 as required. The removable media 21 can also store various data stored in the storage section 18 in the same way as the storage unit 18.

Although not illustrated, the collection device CO, the receptor information determination device 2, the content playback device RE, the display device TV, and the scent spraying device 3 have the hardware configuration illustrated in FIG. 4.

### <Functional Configuration>

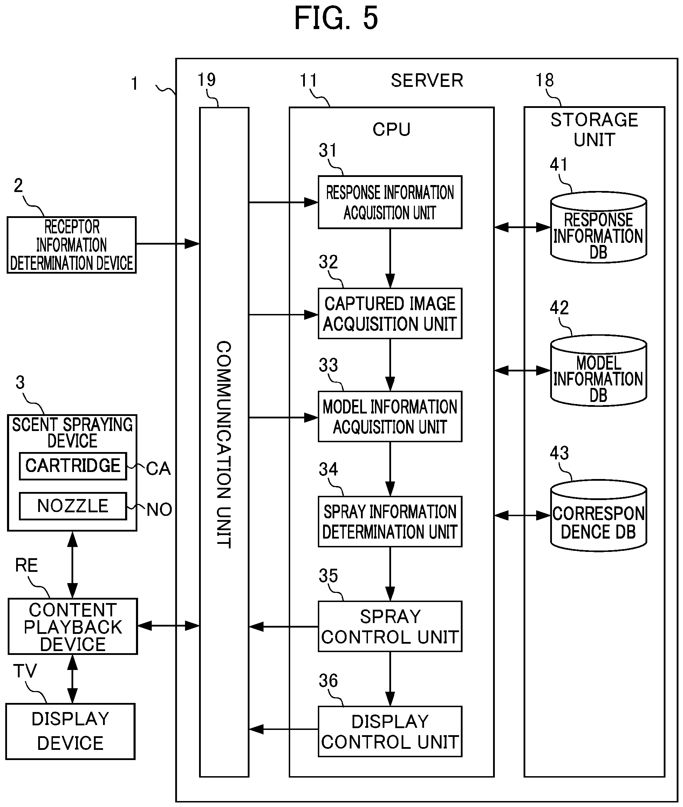

FIG. 5 is a functional block diagram illustrating an example of the functional configuration in the server 1 according to the present embodiment.

### <<Server 1»

A response information acquisition unit 31, a captured image acquisition unit 32, a model information acquisition unit 33, a spray information determination unit 34, a spray control unit 35, and a display control unit 36 function when the CPU 11 of the server 1 operates.

The response information acquisition unit 31 acquires receptor response information obtained using multiple types of receptors, including olfactory receptors (or taste receptors) that are responsive to extracellular substances. More specifically, the response information acquisition unit 31 acquires such response information analyzed by using the receptor information determination device 2. Note that the acquired response information (receptor response information) is stored in a response information DB41. Here, the response information is stored in conjunction with the content (or time information of the content) described above. In this way, for example, the target scent can be reproduced at an appropriate time of the content.

The captured image acquisition unit 32 acquires content that includes at least one of audio information and/or video information. The content is, for example, if capturing information in real time, image information captured by the video camera illustrated in FIG. 1. In addition, if not capturing information in real time, the content may be content broadcast by a terrestrial method or a satellite broadcast, or content broadcast from various distribution servers. If only reproduction of the target scent is performed, the acquisition of various content by the captured image acquisition unit 32 need not be performed.

The model information acquisition unit 33 acquires information on the model of the display device TV, the scent spraying device 3, and the like. With this configuration, for example, spray information suitable for the scent spraying device 3 can be determined in the process of determining the spray information (described below). More specifically, the spray information can be determined according to the type of the cartridge CA of the scent spraying device 3. The acquired model information is stored in a model information DB42. The model information acquisition unit 33 may also acquire information on fragrances (cartridges). This makes it possible, for example, to refer to a table (or learning model) corresponding to the cartridge (type) for determining correspondence between the receptor information and the spray information in the process of determining the spray information to be described below.

The spray information determination unit 34 determines the spray information for scent spraying for multiple types of fragrances according to the response information.

For example, the spray information determination unit 34 determines the spray information based on the response information described above and a predetermined correspondence table (not shown). Note that, it is assumed that the response information and the spray information are previously associated with each other in the correspondence table and stored in a correspondence DB43. Here, for example, it is assumed that the response information and the spray information are correlated in the following manner in the correspondence table.
- A higher "response strength" indicates greater "fragrance spray amount"
- A higher "area (integral value) of response intensity" indicates greater "fragrance spray amount"
- A higher "response durability" indicates longer "fragrance spray time"
- A faster "response time" indicates higher "fragrance spray temperature"
- A faster "peak time" indicates higher "fragrance spray temperature"
- A higher "response rise" indicates higher "fragrance spray temperature"
- A higher "number of peaks" indicates higher "number of fragrance sprays"
- A higher "response strength" indicates smaller "spray port aperture"
Note that the following correlation between response information and spray information are examples and are not limited to the following and may be combined. For example, a higher "response speed" and "response rise" may indicate higher "fragrance spray temperature", for example.

For example, the spray information determination unit 34 determines the spray information based on the response information using a learning model. The learning model is a machine-learning model that outputs the spray information from the response information by learning the correspondence between the receptor response information and the spray information corresponding to the receptor response information using a set of learning data (training data). In the present embodiment, the learning model is stored in the correspondence DB43. The learning method used in the machine learning is not limited and may be unsupervised learning, reinforcement learning, deep learning, or the like. Multiple learning methods may also be combined.

Note that the spray information determination unit 34 may determine the spray information by considering the model information described above. In other words, the spray information determination unit 34 may determine the spray information using the correspondence table or the learning model according to the number (type) of cartridges CA included in the model information.

In addition to the response information described above, the spray information determination unit 34 may also determine the spray information for scent spraying according to spatial parameters. Spatial parameters are parameters related to the state of a space where the scent is to be sprayed or the positional relationship between the scent and the object (subject or object) to be sprayed with the scent. More specifically, the spatial parameters are various parameters such as the size of the room or space where the scent is to be sprayed, the distance between the scent spraying device 3 and a nose, air volume, air direction, temperature, and humidity. The spatial parameters may be acquired by a predetermined acquisition unit (not shown) from spatial parameters sensed by sensors in the scent spraying device 3 or another external device. For example, when the position of a person's nose (face) can be identified to some extent, such as in a movie theater (for direct spraying), the spray information determination unit 34 may control the spray direction, spray volume, spray density and the like based on the distance to the nose, temperature, humidity and the like. For example, in the case of a large space such as a sports stadium (for spraying indirectly), the spray information determination unit 34 may control the spray direction, spray volume, spray concentration and the like based on the size of the space, air volume, air direction, temperature, humidity and the like. The environmental parameters and the spray information may be corresponded with each other using a correspondence table as described above, or by a learning model.

The spray control unit 35 (emission control unit) causes at least one of the multiple types of fragrances (multiple types of cartridges CA) to be sprayed based on the spray information. In other words, the spray control unit 35 sends the spray information to the scent spraying device 3 (display unit TV).

The display control unit 36 transmits the content described above to the display unit TV. The display control unit 36 associates the content with identification information of the spray information. Thus, for example, it is possible to perform multiple sprays of multiple types of target scents at multiple times during the display of the content.

### <<Scent Spraying Device 3>>

As described above, the scent spraying device 3 may have a configuration including a mounting section and an emission port (nozzle NO), and in the CPU (not illustrated) of the scent spraying device 3, an emission control unit (emission control means) may function when the scent spraying device 3 is in operation.

The emission control unit is a functional unit for emitting at least one of a plurality of types of substances related to scent or taste reception from the cartridge CA based on emission information related to the emission of a plurality of types of substances related to scent or taste reception.

The emission control unit may also determine whether the cartridge CA meets a predetermined criterion (authenticity determination). For example, the emission control unit may perform the above determination by acquiring information in an IC chip provided in the cartridge CA. Here, the cartridge CA can be removably attached to the mounting section of the scent spraying device 3 and includes a case that houses a plurality of types of substances related to scent or taste reception, the IC chip, and an interface for exchanging data between the IC chip and the emission control unit. The IC chip includes a non-volatile memory for storing data and a computation device for control processing. The reason for this configuration is that when the scent spraying device 3 reproduces a scent, there is a risk that the appropriate scent (or taste) may not be reproduced if the fragrance in the cartridge CA is not of the intended quality, such as if it has deteriorated over time or is reused and filled with a fragrance that does not meet a prescribed standard. For example, if a predetermined number of days (e.g., six months) have elapsed since the time at which the cartridge CA is first used (e.g., at the time of manufacture, time of installation, time of initial specification, etc.), the emission control unit may alert the user that the cartridge CA has deteriorated over time, output a message encouraging replacement of the cartridge CA, or restrict the use of the scent spraying device 3. The time at which the cartridge CA is first used is stored in the IC chip provided in the cartridge CA, as described above. Note that the non-volatile memory of the IC chip in the cartridge CA may store identification information of the cartridge CA, and the identification information and information such as the time at which the cartridge CA is first used associated with the identification information may be stored in the storage unit 18 in the server 1 described above. Also note that the emission control unit may update information such as the time at which the cartridge CA is first used in a case where the cartridge CA meets the predetermined criterion.

The emission control unit may also adjust the emission information according to the state of use of the cartridge CA. For example, when using a cartridge that has been in use for a predetermined period of time, the emission control unit may spray a larger amount of fragrance from the cartridge CA than usual or increase the spray time.

### <Processing Content>

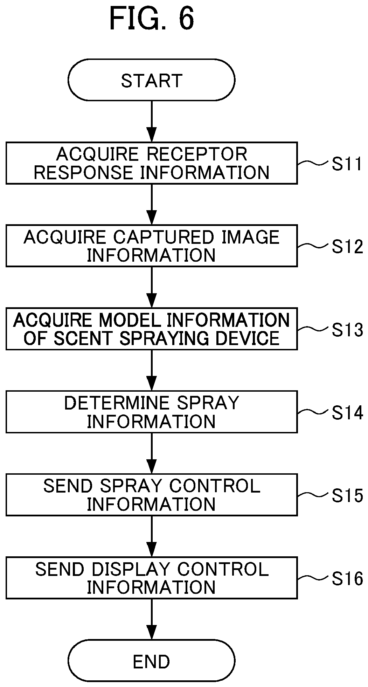

FIG. 6 illustrates an example of a spray control process according to the present embodiment. In the present embodiment, an example in which the spray control process is performed by the server 1 is described, but no limitation is intended. For example, the spray control process may be performed by the scent spraying device 3.

In step S11, the response information acquisition unit 31 acquires the response information of the receptor corresponding to the target scent. In step S12, the captured image acquisition unit 32 acquires video information as the content. In step S13, the model information acquisition unit 33 acquires the model information and the like of the scent spraying device 3. In step S14, the spray information determination unit 34 determines the spray information related to scent spraying for multiple types of fragrances according to the response information. In step S15, the spray control unit 35 sends the spray information to the scent spraying device 3 (display unit TV). In step S16, the display control unit 36 transmits the content to the display unit TV.

### <Advantageous Effects of This Embodiment>

According to the embodiment described above, a fragrance can be sprayed in correspondence with the temporal position (time axis) of the content during content playback (viewing). This allows a user to view the content using not only their sense of sight and hearing but also their sense of smell, thereby improving immersion in the content and a sense of affinity for the shooting location. In addition, for example, VR goggles (HMD) can function as the scent spraying device to further enhance the immersive experience.

In addition, according to the embodiment described above, by releasing the target scent in synchronization with the video, for example, an advertising effect can be expected. For example, a display-type device equipped with a scent spraying function can be prepared outdoors, and by having customers select a predetermined menu (by having them select a button), a scent appropriate for a certain dish can be instantly reproduced and sprayed along with an image of the dish. The manner of reproduction can be changed to correspond to various food scents.

In addition, according to the embodiment described above, it is possible to communicate with a friend in a remote location via smell. For example, when describing an experience, smells in addition to words, gestures, videos and photos can be incorporated as elements to add depth to the shared experience. This allows for smoother and deeper communication.

The embodiment described above can also be used for a presentation. For example, when a product is developed, it may be explained in an online meeting. Fragrance can be an important factor, especially for food and cosmetics. However, it is difficult for people in remote areas to smell the product directly. Therefore, by adopting the embodiment described above, it is possible to share smells with people on the other side of the screen who are in a remote location, in a manner similar to sharing a screen. This is expected to increase the probability of success in business negotiations.

An embodiment of the invention has been described above, but the invention is not limited to the embodiment described above, and variations, improvements and the like are included in the invention to the extent that the purpose of the invention can be achieved.

### (Modifications)

In the embodiment described above, the nozzle NO sprays a fragrance according to the spray time and other factors. The nozzle NO may spray the fragrance in a direction where people are present based on information acquired by a predetermined motion sensor. When outdoors, a wind direction sensor may be used to determine the spray direction according to the wind direction. This makes it possible to reproduce the target scent with greater accuracy for the user viewing the content.

The embodiment described above is an example of reproducing a target scent associated with content, but no limitation is intended. For example, a predetermined smell (e.g., "the smell of soy sauce ramen") may be singled out, acquired from a predetermined storage (library), and reproduced. This allows for the reproduction of rare natural scents by means of substituted scents (cartridge CA), or toxic scents by means of non-toxic scents, for example. This can also help the user relax by using pre-set "calming scents", "concentration-enhancing scents", "sleep-enhancing scents", and the like (using response information prepared in advance).

While the embodiment described above does not have an alert function based on smell, the server 1 may function as an infection checker, cancer checker, Parkinson's disease checker, or health care toilet based on smell. For example, a scent can be collected and reproduced by a remote medical professional or other person for diagnostic use.

The embodiment described above is an example of generating response information by olfactory receptors or taste receptors in a human, but the response information may also be generated by receptors of animals other than humans. For example, the embodiment can also be applied to a repellent (e.g., insect repellent) or an attractant. This also allows the embodiment to be applied to pest control.

In the embodiment described above, an example is described of reproducing the target scent at a timing corresponding to the content, but the scent may be sprayed at various timings. For example, a request may be sent from the scent spraying device 3 to the server 1 in response to a user operation, and upon acceptance of the request, the spray control unit 35 may send spray information. Then, upon acceptance of the spray information, the scent spraying device 3 may reproduce the target scent. For example, the scent spraying device 3 may accept the spray information in advance and reproduce the target scent by controlling the scent spraying device 3 in response to a user operation.

The embodiment described above is an example in which the spray control unit 35 transmits the spray information, but the spray control unit 35 may also manage the number of sprays based on a maximum number of sprays related to the spraying of the target scent. For example, the spray control unit 35 may determine whether to send the spray information based on the maximum number of sprays and a number of completed sprays, which are stored in a predetermined storage unit. Note that the maximum number of sprays and the number of completed sprays described above may be managed by the scent spraying device 3. In this case, the spray control unit 35 transmits the maximum number of sprays together with the spray information. The scent spraying device 3 may then determine whether to send the spray information based on the maximum number of sprays and the number of completed sprays, which are stored in a predetermined storage unit.

In the embodiment described above, scent spray control is performed in which a fragrance is sprayed (emitted) to reproduce a target scent, but the embodiment can also be applied to flavor emission control in which a seasoning is emitted to reproduce a target taste. In other words, information such as response intensity, area of response intensity, response durability, response speed, peak time, response rise, and number of peaks is input as response information for taste instead of response information for scent molecules as described above. In addition, instead of the scent spray information described above, information such as emission time, emission amount, emission temperature, emission frequency, and emission port aperture for substances related to taste reception (e.g., seasonings) are output as taste emission information.

In addition, the embodiment described above is an example of reproducing a scent (or taste) when playing back video content, but the embodiment can also be applied in a virtual space such as a so-called "metaverse". For example, when traveling in a virtual space using an avatar (domestic travel, overseas travel, space travel, etc.), a perfume or other scent may be emitted in accordance with the avatar's words and actions. For example, a scent code of a predetermined perfume may be converted into a Non-Fungible Token (NFT) using blockchain technology and enabled as a copyright or distributed as a virtual currency. With this configuration, in the distribution of products with scent and taste (e.g., perfumes), actual transportation of the product can be eliminated, and the product can be delivered to the consumer immediately.

In the embodiment described above, response information is converted into spray information based on a correspondence table and using a learning model, but the response information may be sent as is without using these. In this case, the scent spraying device 3 may reproduce the scent to some extent by using the cartridge CA that corresponds one-to-one to the receptor OR. This allows for different usage depending on the situation, such as sending response information to a low-cost version of the scent spraying device 3 without using a correspondence table or the like.

### (Second Embodiment)

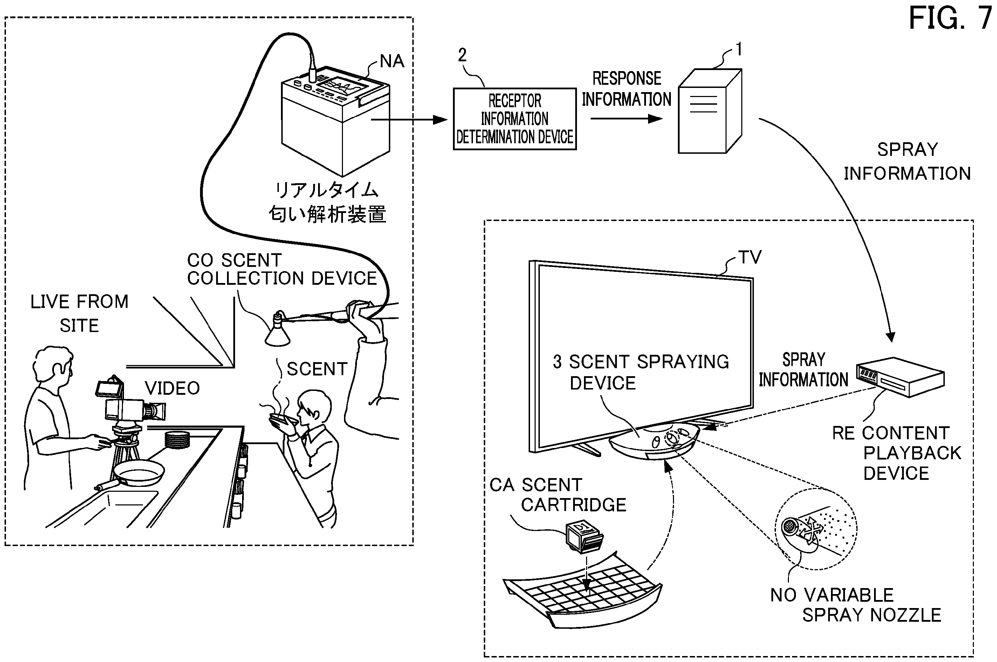

FIG. 7 illustrates an overview of a scent reproduction method to which an information processing device according to a second embodiment is applied. FIG. 7 illustrates an example in which content introducing a food or beverage (e.g., ramen noodles) at a restaurant is distributed to the viewer (in real time), and the smell of the food or beverage is reproduced on the viewer's side using a predetermined device. Examples of the content include content distributed via terrestrial broadcasting, satellite broadcasting, and the Internet.

In the example illustrated in FIG. 7, a predetermined camera captures an image of visitors consuming food or beverages at a restaurant, and a scent collection device CO collects the scent (e.g., gas) of the food or beverage. The collected scent is analyzed by a real-time scent analysis device NA (gas detection device U100) and converted into response information for a predetermined receptor by the receptor information determination device 2 (conversion device). The converted response information is sent to the server 1. Then, spray information is sent together with the content from the server 1 to each content playback device RE. The content playback device RE transmits the content to a predetermined display device TV based on a request to view the content. The content playback device RE also sends spray information corresponding to the content to the scent spraying device 3. The scent spraying device 3 includes multiple types of scent cartridges CA, and fragrances (e.g., liquid or powder) extracted from the multiple types of scent cartridges CA are sprayed (ejected, jetted) via variable spray nozzles (nozzles NO).

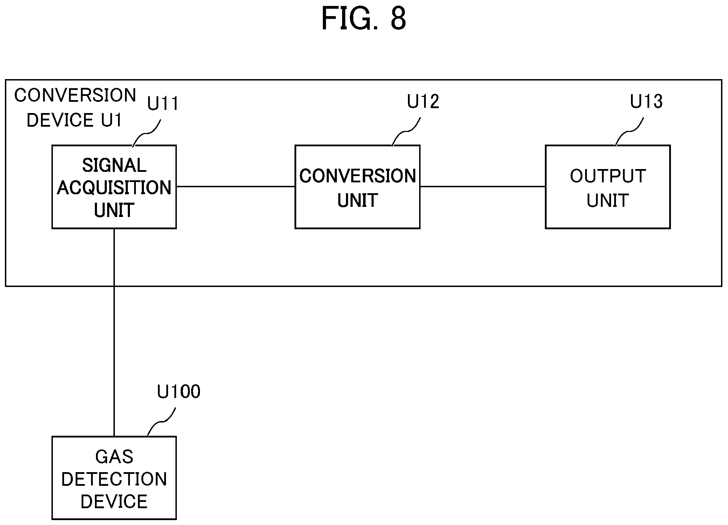

The receptor information determination device 2 (conversion device) in FIG. 7 will be explained with reference to FIGS. 8 to 16. First, an overview of a conversion device U1 is described prior to describing the conversion device U1, a prediction model creation device U2, a conversion information creation method, a prediction model creation method, and a program of each embodiment.

First, the purpose of using the conversion device U1 is explained. The use of gas sensors has been proposed as a system to identify the type of a scent. However, it is difficult to accurately determine and identify the type of a scent perceived by humans using only gas sensors. This is because the sensing element of the gas sensor is composed of a substance that is not in line with human senses (sense of smell). Thus, even though various detection elements and analysis methods have been proposed, it is difficult to identify and determine the type of a scent.

While some approaches forcibly link information from gas sensor output and human sensory testing through machine learning or other means, highly accurate judgments based on the above approaches have not yet been achieved because of the variability in the evaluation of sensory test data between individuals. Some approaches, such as a notation method called SMILES notation that uses alphanumeric strings of molecular chemical structures, can be used to strengthen the link between gas sensor output and sensory test results. However, although this approach improves prediction accuracy, highly accurate identification, determination, and prediction of scent types could not be achieved due to the inclusion of information related to molecules with no scent. On the other hand, if the gas sensor output can be expressed in terms of a quantitative human olfactory value, it is possible to determine scent with high accuracy and in accordance with the human sense of smell.

The conversion device U1 is, for example, a device that converts output information output from a device with a predetermined gas detection function for a predetermined scent molecule (hereinafter also referred to as gas detection device U100) into response information indicating the response of the olfactory receptor. With this configuration, the conversion device U1 converts the output information of different values output from each of the gas detection devices U100 with different analysis methods for the same scent molecules into a single piece of response information, which is the response of the olfactory receptor U200 that yields the same result. This allows the conversion device U1 to link the output information of the gas detection device U100 to a single piece of response information, regardless of the different analysis methods of the gas detection device U100. For example, the conversion device U1 can link one piece of response information to the output information with different values and units for each of several gas detection devices U100 for one scent molecule, as a so-called scent meter bar. In the following embodiment, the conversion device U1 converts the output information into the response information using a learning model already subjected to machine learning.

Next, the gas detection system U100 will be described. The gas detection device U100 is, for example, a gas sensor or a mass spectrometer (gas chromatograph-mass spectrometer). Depending on the type and concentration of predetermined scent molecules, the gas sensor outputs, as the output information, a predetermined electrical signal (e.g., voltage value, resistance value, or current value), frequency change, light wavelength change, or activation level due to exposure of the scent molecules to olfactory receptors. For example, the gas detection device U100 outputs an electrical signal with a larger voltage value the greater the magnitude of the concentration of the scent molecules that come into contact with the gas detection device U100. The mass spectrometer outputs, as graph data, for example, a plot showing mass charge and its strength with respect to the type and concentration of the scent molecules.

Next, the olfactory receptor U200 will be described. The olfactory receptor U200 can be realized, for example, by using the known olfactory receptors described in Patent Document 1 and the like. The olfactory receptor U200 is, for example, a nucleic acid that is mounted in contact with a substrate (not shown). Nucleic acids include nucleic acids containing genes encoding a predetermined receptor. There are multiple types of nucleic acids in contact with the substrate, and various types of nucleic acids are placed on the substrate separated from each other. By bringing cells into contact with the nucleic acids on the substrate, the cells transiently expressing the olfactory receptors corresponding to the various nucleic acids are generated in situ.

When the cell is brought into contact with a test substance, receptors in this form can cause changes in the cellular state. Specifically, changes in intracellular calcium concentration or intracellular cAMP concentration can occur. Such changes can be measured by using a cAMP-sensitive dye, a cAMP-sensitive fluorescent protein, a calcium-sensitive dye, or a calcium-sensitive fluorescent protein. For example, the degree of receptor activation can be quantitatively calculated by measuring the change in brightness caused by the cAMP-sensitive dye or the cAMP-sensitive fluorescent protein. The degree of activation is used as response information indicating the response of the olfactory receptor U200 or response information.

The olfactory receptor may be a human, mammalian, insect, or nematode olfactory receptor. The olfactory receptor is not limited to a liquid test substance and may also be in contact with and respond to a gaseous (gaseous) test substance. The latter form of the olfactory receptor can be used as either the gas detection device U100 or the olfactory receptor U200. In other words, it is also possible to convert between the olfactory receptor response information for a gaseous test substance and the olfactory receptor response information for a liquid test substance.

The olfactory receptor is not limited to the form expressed a cell described above and may also have a cell-free form. For example, liposomes formed by lipid bilayers, such as cell membranes, with various olfactory receptors on the membranes, may be arranged isolated from each other on the substrate. The size of the liposomes is not limited and may typically be around 100 nm in diameter. The manufacturing process for the liposomes is not limited. The cells expressing olfactory receptors may be divided into a membrane fraction (which may contain intracellular signaling proteins such as G proteins, adenylate cyclase, and cyclic nucleotide-dependent channels) and a cytosolic fraction (which may contain intracellular signaling substances such as GDP, GTP, ATP, and cAMP), and both fractions may be mixed and stirred to combine the fractions. Alternatively, the olfactory receptor protein itself may be used as a substrate probe. Here, nanodiscs that retain the three-dimensional structure of olfactory receptors penetrating the cell membrane are suitable. Nanodiscs, for example, are membrane scaffold proteins (MSPs) made up of a mutant of apolipoprotein A1 (APOA1) and can accumulate lipid bilayers in the form of discs (Timothy H. Bayburt, Yelena V. Grinkova, and Stephen G. Sligar Nano Letters 2002 2 (8), 853-856). Thus, nanodiscs can retain membrane proteins outside the cell while penetrating the lipid membrane (Civjan NR, Bayburt TH, Schuler MA, Sligar SG. Direct solubilization of heterologously expressed membrane proteins by incorporation into nanoscale lipid bilayers. Biotechniques. 2003 Sep; 35(3) : 556-60, 562-3). The substrate on which the nanodiscs are placed is not limited and may be a carbon nanotube FET (Yang H, Kim D, Kim J, Moon D, Song HS, Lee M, Hong S, Park TH. Nanodisc-Based Bioelectronic Nose Using Olfactory Receptor Produced in Escherichia coli for the Assessment of the Death-Associated Scent Cadaverine. ACS Nano. 2017 Dec. 26; 11(12): 11847-11855. doi: 10.1021/acsnano) or a carbon nanotube FET. The manufacturing process for the nanodiscs is not limited and may include a process of forming nanodiscs by self-assembly by mixing solubilized membrane proteins, which are MSP expressed and recovered in E. coli or other bacteria, and phospholipids dissolved in water with a surfactant, and then removing the surfactant by dialysis or other means. By using these cell-free forms of olfactory receptors, the degree of receptor activation can also be quantitatively evaluated by measuring changes in current, voltage, impedance and the like caused by the test substance.

Next, the conversion device U1, a method for creating conversion information, and a program according to the second embodiment of the present invention will be described with reference to FIGS. 8 to 12. In the present embodiment, a gas sensor is described as an example of the gas detection device U100.

The conversion device U1 converts the output information output from the predetermined gas detection device U100 for a predetermined scent molecule into response information indicating the response of the olfactory receptor U200. The conversion device U1 according to the present embodiment outputs the converted response information and also outputs words (phrases) representing the predetermined scent characteristics from the converted response information. As illustrated in FIG. 8, the conversion device U1 includes an output information acquisition unit U11, a conversion unit U12, and an output unit U13.

The output information acquisition unit U11 is realized, for example, by a CPU operating. The output information acquisition unit U11 acquires output information from the gas detection device U100 for a predetermined scent molecule. The output information acquisition unit U11, for example, acquires a voltage value output from the gas detection device U100 upon sensing a predetermined scent (scent molecules) as the output information. The output information acquisition unit U11 may, for example, acquire output information measured remotely by the gas detection device U100.

The conversion unit U12 is realized, for example, by a CPU operating. The conversion unit U12 converts the acquired output information into response information based on output signals output from the plurality of gas detection devices U100 for each of the plurality of scent molecules and the response information indicating the response of the olfactory receptors U200 to each of the plurality of scent molecules. In the present embodiment, the conversion unit U12 converts the acquired output information into response information indicating the response of the olfactory receptor U200 using a machine learned prediction model in which the output information output from the plurality of gas detection devices U100 for each of the plurality of scent molecules is an explanatory variable and the response information indicating the response of the olfactory receptors U200 to each of the plurality of scent molecules is an object variable.

The conversion unit U12 uses a machine-learned predictive model which uses, as the explanatory variable, variables newly created by a numerical, functional, spatial or time-series index, or feature engineering, calculated using mathematical, statistical, or machine learning methods on the output information output from the gas detection device U100. The conversion unit U12 uses a machine-learned predictive model which uses, as the object variable, variables newly created by a numerical, function, spatial or time-series index, or feature engineering, calculated using mathematical, statistical, or machine learning methods of the response information of the olfactory receptors U200. For example, the conversion unit U12 uses a machine-learned prediction model in which time-series data and the number of feature patterns of the output information output from the plurality of gas detection devices U100 are used as explanatory variables and time-series data and the number of feature patterns of the response information of the plurality of olfactory receptors U200 is used as object variables to convert the acquired output information into response information.

In the present embodiment, the conversion unit U12 uses a machine-learned prediction model in which the presence or absence of the output information from the plurality of gas detection devices U100 is the explanatory variable. In the present embodiment, the conversion unit U12 uses a machine-learned prediction model in which the presence or absence of response of the plurality of receptors in the olfactory receptors U200 is the object variable. In the present embodiment, the conversion unit U12 uses a machine-learned predictive model in which the mass charge and strength of the output information output from the gas detection devices U100 is the explanatory variable. In this way, the conversion unit U12 converts the output information to the response information indicating the degree of activation of the olfactory receptors U200 for the voltage value for conversion indicated by a voltage value, resistance value, current value, frequency change, or light wavelength change.

The output unit U13 is realized, for example, by a CPU operating. The output unit U13 outputs the converted response information. The output unit U13 outputs the response information by displaying it, for example. For example, the output unit 13 outputs a numerical value of the activation level as the response information.

Next, the program of the present embodiment will be described. Each of the components in the conversion unit U1 can be realized by hardware, software, or a combination of the two, respectively. Here, "realized by software" means that a component is realized by a computer reading and executing a program.

The program can be stored using various types of non-transitory computer readable media (non-transitory computer readable medium) and can be supplied to a computer. Examples of the non-transient computer readable media include various types of tangible storage media. Examples of the non-transient computer readable media include magnetic recording media (e.g., flexible disks, magnetic tape, and hard disk drives), magneto-optical recording media (e.g., magneto-optical disks), CD-ROMs (read only memory), CD-Rs, CD-R/Ws, and semiconductor memories (e.g., mask ROM, programmable ROM (PROM), erasable PROM (EPROM), flash ROM, and random-access memory (RAM)). The display program may also be supplied to the computer by various types of transitory computer readable media (transitory computer readable medium). Examples of the transitory computer readable media include electrical signals, optical signals, and electromagnetic waves. The transitory computer readable media can supply the program to a computer via wired or wireless communication paths, such as wire and optical fiber.

Next, examples of the present embodiment will be described.

### (Example 1)

An array sensor including 400 olfactory receptors was used as the olfactory receptor U200. A predetermined scent was then exposed to olfactory receptor-expressing cells for a predetermined amount of time. The concentration of scents ranged from 500 µM to 10 µM. Output information was acquired by exposing 12 different gas detection devices U100 (gas sensors) to a predetermined scent for a predetermined period of time. The conversion device U1 was then used to convert the output information to response information. The degree of activation resulting from exposure to expressing cells of the olfactory receptor U200 was also measured. Here, as the measured scent molecules, hexylacetate, hexylbutyrate, butylbutyrate, 2,7-octadienol, cis-2-penten-1-ol, toluene, beta-ionone, benzothiazole, cyclotene, acetic acid, coumarin, 1,2,4 trimethyl benzene, 2-ethylhexanol, propionaldehyde, 4-Isopropylphenol, bis(methylthio)methane, 1,2,4,5 -tetramethyl benzene, 3-methyl-1-butanol, E-2-nonenal, and m-cresol were used.

For data processing, python was used as the language for the prediction model. In addition, random forest, support vector machine, and gradient-boosting decision trees were each used as algorithms for evaluation by the prediction model. As the prediction model, a model produced by regression was used. Supervised learning was used as the learning method. The model evaluation method was mainly based on the coefficient of determination.

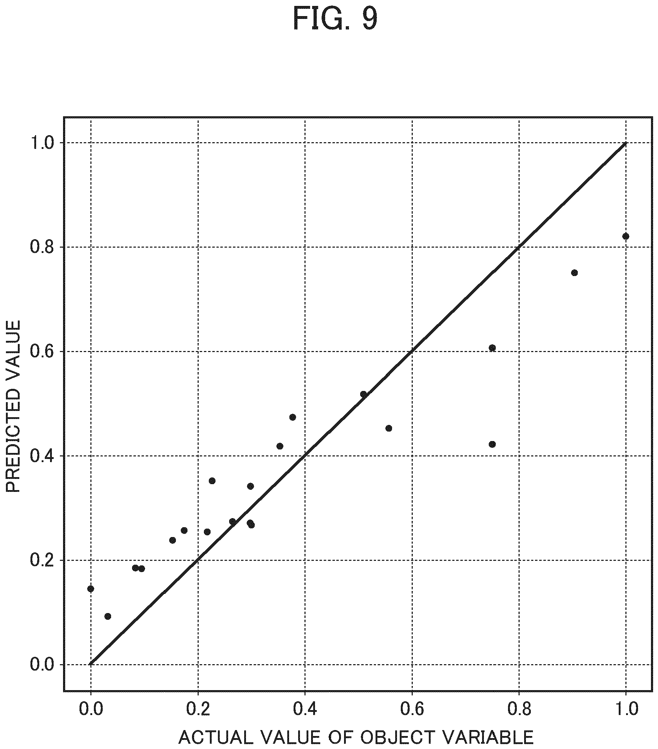

When regression using the random forest method was used, a regression coefficient of determination of 0.834 (with a maximum of 1.0) was obtained, as illustrated in FIG. 9, with the vertical axis as the predicted value (response information) output from the prediction model and the vertical axis as the actual value of the object variable (the degree of activation of the expressing cells of the olfactory receptor U200) .

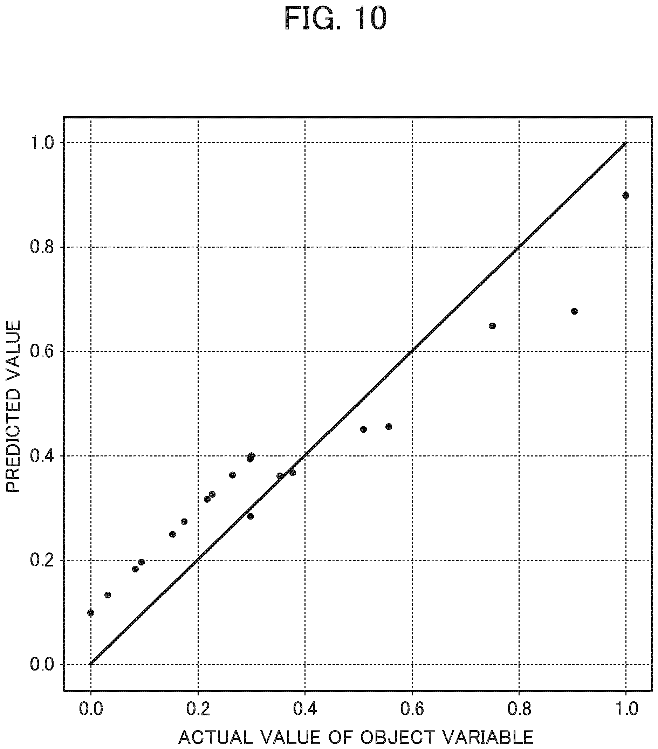

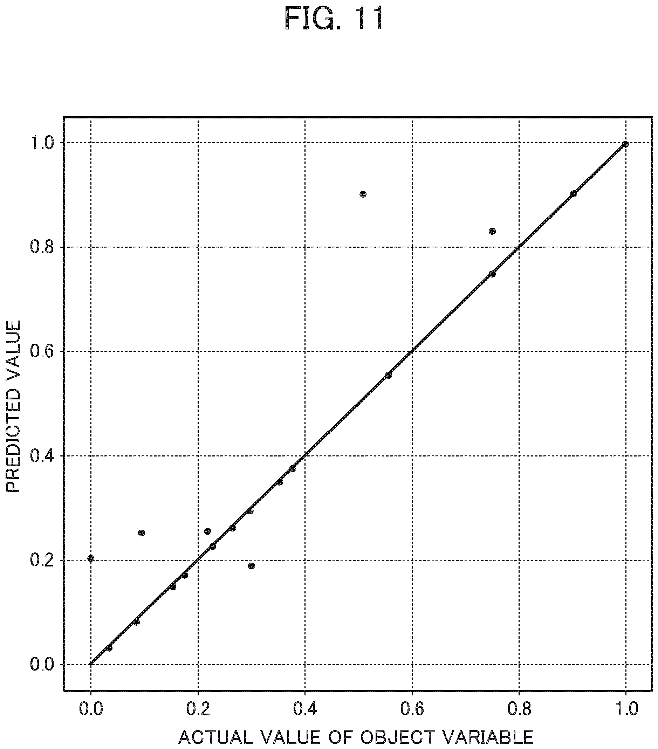

When using regression using a support vector machine, a regression coefficient of determination of 0.871 was obtained, as illustrated in FIG. 10. When using regression using a gradient-boosted decision tree, a regression coefficient of determination of 0.847 was obtained, as illustrated in FIG. 11. This made it possible to obtain sufficient correlation of the predicted values with the actual values of the object variable. In other words, it was found that the output information is quantifiable.

### (Example 2)

A model that learns sensor data for predetermined scent molecules and predicts the responding olfactory receptor U200 and its degree of activation was created. Test conditions and test procedures for the gas detection system U100 and the olfactory receptor U200 cell array sensor were the same as in Example 1. Class classification was used in the data processing. Python was used as the language. A neural network was used as the algorithm. To relate the output information of the gas detection device U100 to the response information of the olfactory receptor U200, a class classification model was created. The learning method was supervised learning. While any number of hidden layers can be used, two layers were used in this example. The model was evaluated using an overall percentage of correct responses "accuracy" and a loss function "loss". The output information of the gas detection device U100 was used as the explanatory variable, and the response information indicating the response of the olfactory receptor U200 was used as the object variable.

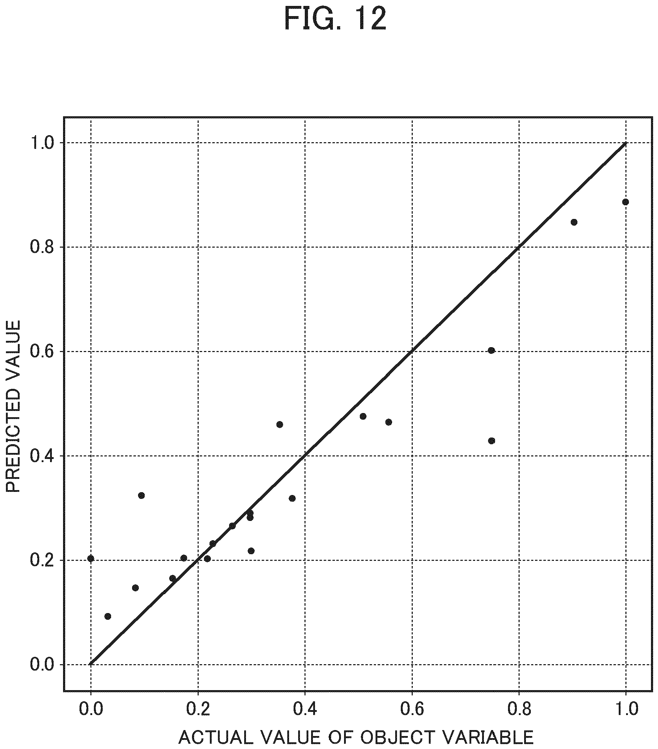

As illustrated in FIG. 12, the predicted "accuracy" is maintained high at around 0.9 and the "loss" is maintained low at around 0.3 after about 500 learning cycles. From this, we were able to produce the prediction model described above.

According to the conversion device U1, the conversion information creation method, and the program of the first embodiment, the following effects are achieved.

(1) The conversion device U1 is a conversion layer subject configured to convert output information output from a predetermined gas detection device U100 for predetermined scent molecules into response information indicating the response of the olfactory receptor U200, and includes the output information acquisition unit U11 configured to acquire output information output from the gas detection device U100 for predetermined scent molecules, the conversion unit U12 configured to convert the acquired output information into response information based on the output signals output from the plurality of gas detection devices U100 for each of the plurality of scent molecules and the response information indicating the response of olfactory receptor U200 for each of the plurality of scent molecules, and the output unit U13 configured to output the converted response information. This allows output information obtained from various gas detection devices U100 to be converted into one type of response information. Thus, it is possible to correlate between gas detection devices U100.
(2) The conversion unit U12 converts the acquired output information into response information indicating the response of the olfactory receptor U200 using a machine learned prediction model in which the output information output from the plurality of gas detection devices U100 for each of the plurality of scent molecules is an explanatory variable and the response information indicating the response of the olfactory receptors U200 to each of the plurality of scent molecules is an object variable
   Thus, using machine learning can further improve conversion accuracy.
(3) The conversion unit U12 uses a machine-learned predictive model which uses, as the explanatory variable, variables newly created by a numerical, functional, spatial or time-series index, or feature engineering, calculated using mathematical, statistical, or machine learning methods on the output information output from the gas detection device U100. This can improve the accuracy of the conversion.
(4) The conversion unit U12 uses a machine-learned predictive model which uses, as the object variable, variables newly created by a numerical, function, spatial or time-series index, or feature engineering, calculated using mathematical, statistical, or machine learning methods of the response information of the olfactory receptors U200. This can improve the accuracy of the conversion.
(5) The conversion unit U12 uses a machine-learned prediction model in which time-series data and the number of feature patterns of the output information output from the plurality of gas detection devices U100 are used as explanatory variables and time-series data and the number of feature patterns of the response information of the plurality of olfactory receptors U200 is used as object variables to convert the acquired output information into response information. This can improve the accuracy of the conversion.
(6) The conversion unit U12 uses a machine-learned prediction model in which the presence or absence of the output information from the plurality of gas detection devices U100 is the explanatory variable. This can improve the accuracy of the conversion.
(7) The conversion unit U12 uses a machine-learned prediction model in which the presence or absence of response of the plurality of receptors in the olfactory receptors U200 is the object variable. This can improve the accuracy of the conversion.

### (Third Embodiment)

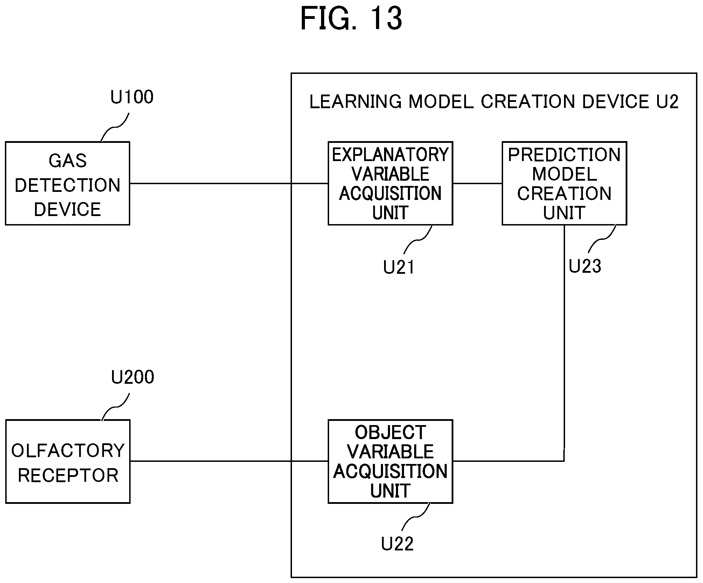

Next, the prediction model creation device U2, a prediction model creation method, and a program according to a third embodiment of the present invention will be described with reference to FIG. 13. In describing the third embodiment, the same configurations as in the above-described embodiments are denoted by the same reference signs and descriptions thereof are omitted or simplified. The prediction model creation device U2, the prediction model creation method, and the program according to the third embodiment are a device, method, and program for creating the prediction model according to the first embodiment.

The prediction model creation device U2 creates the prediction model that converts the output information output from the gas detection device U100 into quantified data for predetermined scent molecules. As illustrated in FIG. 13, the prediction model creation device U2 includes an explanatory variable acquisition unit U21, an object variable acquisition unit U22, and a prediction model creation unit U23.

The explanatory variable acquisition unit U21 is realized, for example, by a CPU operating. The explanatory variable acquisition unit U21 acquires the output information output from the plurality of gas detection devices U100 for each of the plurality of scent molecules as explanatory variables. The explanatory variable acquisition unit U21 may use, as output information, the sum of the outputs of the plurality of gas detection devices U100 (e.g., 20 types), mean value, presence or absence of reactions, various indicators, the product of the feature of the output and the number of gas detection devices U100, the product of time series data and the number of feature patterns and the number of gas detection devices U100 or the like.

The object variable acquisition unit U22 is realized, for example, by a CPU operating. The object variable acquisition unit U22 acquires, as the object variable, response information indicating the response of the olfactory receptor U200 to each of the plurality of scent molecules. The object variable acquisition unit U22 may use, as the output information, the total output of the olfactory receptors U200 (e.g., 400 spots), the mean value, the presence or absence of response, various indices, the product of the feature of the output and the number of spots of the olfactory receptors U200, the product of time series data, the number of feature patterns and the number of spots of the olfactory receptor U200 or the like.

The prediction model creation unit U23 is realized, for example, by a CPU operating. The prediction model creation unit U23 creates the prediction model by using the acquired explanatory variables and the acquired object variables in machine learning. The prediction model creation unit U23 creates the prediction model by using correlation coefficients, principal factor analysis, and logistic regression.

Next, the operation of the prediction model creation device U2 (prediction model generation method) will be described. First, the explanatory variable acquisition unit U21 acquires output information as the explanatory variable. Subsequently, the object variable acquisition unit U22 acquires the response information as the object variable. Subsequently, the prediction model creation unit U23 creates a prediction model using the explanatory variable and the object variable.

Next, the program of the present embodiment will be described. Each of the components in the prediction model creation device U2 can be realized by hardware, software, or a combination of the two, respectively. Here, "realized by software" means that the device is realized by a computer reading and executing a program.

Programs can be stored using various types of non-transitory computer readable media (non-transitory computer readable medium) and can be supplied to a computer. Examples of the non-transient computer readable media include various types of tangible storage media. Examples of the non-transient computer readable media include magnetic recording media (e.g., flexible disks, magnetic tape, and hard disk drives), magneto-optical recording media (e.g., magneto-optical disks), CD-ROMs (read-only memory), CD-Rs, CD-R/Ws, and semiconductor memories (e.g., mask ROM, programmable ROM (PROM), erasable PROM (EPROM), flash ROM, and random access memory (RAM)). The display program may also be supplied to the computer by various types of transitory computer readable media (transitory computer readable medium). Examples of the transitory computer readable media include electrical signals, optical signals, and electromagnetic waves. The transitory computer readable media can supply the program to a computer via wired or wireless communication paths, such as wire and optical fiber.

According to the prediction model creation device U2, the prediction model creation method, and the program according to the third embodiment, the following effects are achieved. (7) The prediction model creation device U2 creates a prediction model that converts output information output from the gas detection device U100 for predetermined scent molecules, and includes an explanatory variable acquisition unit U21 that acquires, as an explanatory variable, output information output from the plurality of gas detection devices U100 for each of the plurality of scent molecules, an object variable acquisition unit U22 that acquires, as an object variable, response information indicating the response of the olfactory receptors U200 to each of the plurality of scent molecules, and the prediction model creation unit U23 that creates the prediction model by using the acquired explanatory variable and the acquired object variable in machine learning.

This allows for the creation of a prediction model that converts output information obtained from various gas detection devices U100 into response information. Since the output information can be quantified by being converted into response information, the conversion device U1 can be configured such that correlation between the gas detection devices U100 is possible.

### (Fourth Embodiment)

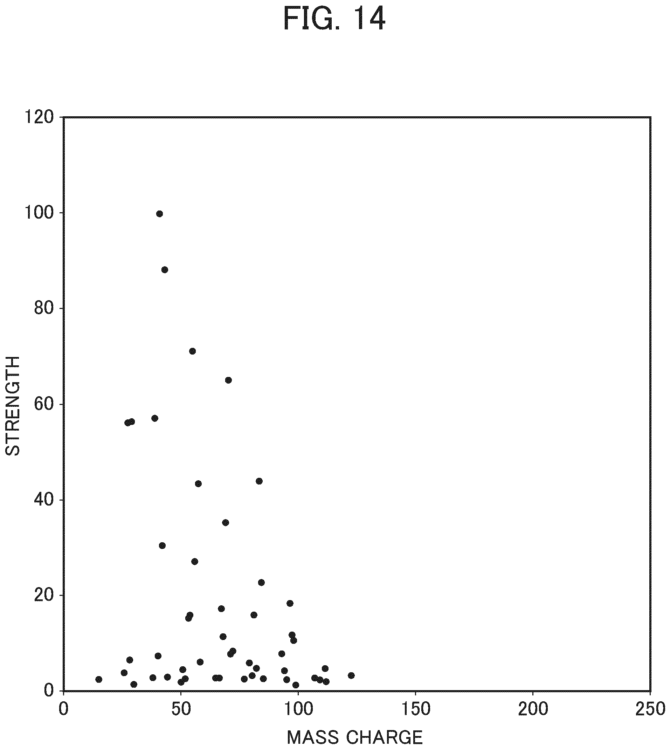

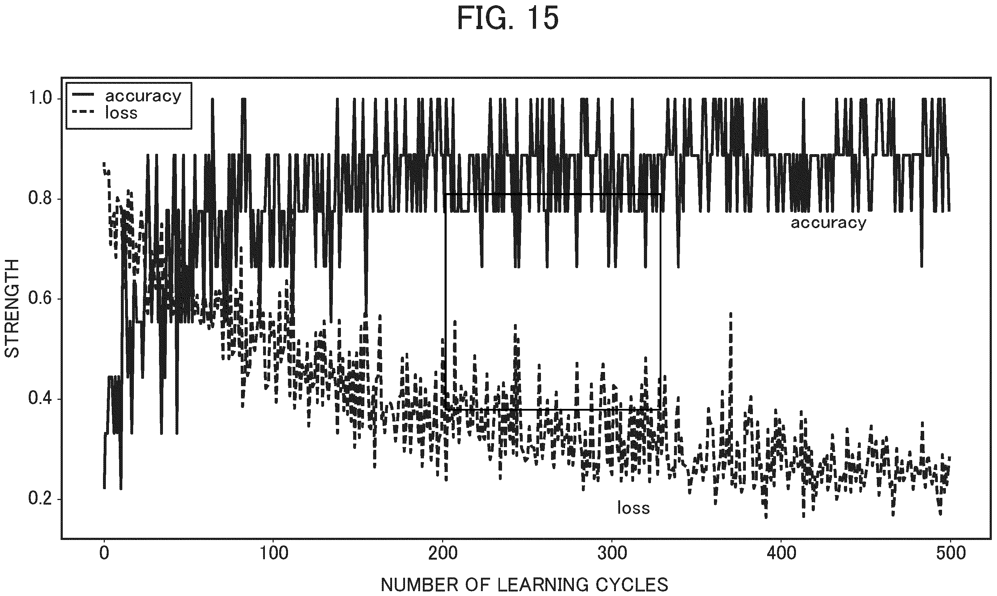

Next, the conversion device U1, a conversion information creation method, and a program according to the fourth embodiment of the present invention will be described with reference to FIGS. 14 and 15. In describing the fourth embodiment, the same configurations as in the above-described embodiments are denoted by the same reference signs and descriptions thereof are omitted or simplified.

The conversion device U1 according to the fourth embodiment differs from that of the first and third embodiments in that the conversion device U1 according to the fourth embodiment uses a mass spectrometer (gas chromatographmass spectrometer) as the gas detection device U100. The conversion device U1 according to the fourth embodiment differs from that of the first and third embodiments in that the output of the gas detection device U100 (mass spectrometer) is used as the output information. The conversion device U1 according to the fourth embodiment acquires, as output information, the set of intensity plots against mass charge output from the gas detection device U100. The conversion device U1 acquires, as output information, a set of intensity plots relative to mass charge obtained as a result of the detection of gaseous compounds by the gas detection device U100, for example. In other words, the conversion device U1 acquires, as output information, data of the mass charge of fragment ions (including noise) generated when gas molecules are ionized, prior to the process of identifying the molecular structure of gaseous compounds by the gas detection device U100. The conversion device U1 converts the output information into the response information of the olfactory receptor U200 by using a prediction model in which the mass charge data (including noise) is used as the explanatory variable. In this way, the conversion device U1 converts the output information into response information of the olfactory receptors, regardless of the characteristics of the equipment, which differ depending on the gas detection device U100. The gas detection device U100 is not limited to detection and may also be a device that performs collection, measurement, and analysis.

Next, an example according to the fourth embodiment will be described.

### (Example 3)

The data acquisition method for the gas detection device U100 was as follows: after diluting a scent molecule solution 1,000-fold with solvent, 1 pL was collected with a syringe and placed in the gas detection device U100. The mass charge data for each molecular type obtained at the time of detection was used for conversion. Test conditions and test procedures for the olfactory receptor cell array sensor were the same as in Example 1.

In data processing, the model was created by random forest regression as in Example 1. The language used was python. The learning method was supervised. The coefficient of determination was used for model evaluation. The data processing of the mass charge acquired by the gas detection device U100 was graphed, and the intensity of each pixel of the image was read, as shown in FIG. 14. The data read was used as output information. The output information of the gas detection system U100 (mass spectrometer) was used as the explanatory variable, and the response information indicating the response of the olfactory receptor U200 was used as the object variable.

As a result, as shown in FIG. 15, a regression coefficient of determination of 0.828 was obtained, with the vertical axis as the predicted value (response information) output from the prediction model and the vertical axis as the actual value of the object variable (degree of activation of the expressing cells of the olfactory receptor U200).

According to the prediction model creation device U2, the prediction model creation method, and the program according to the fourth embodiment, the following effects are achieved. (8) The conversion unit U12 uses the output signal of a mass spectrometer serving as the gas detection device U100 as output information. This allows correlation between the gas detection devices U100 by converting output information to response information for mass spectrometers as well. In particular, by using the mass charge data (including noise) as the output information, the conversion unit U12 can shorten the time from the collection of gaseous compounds to the conversion to response information indicating the response of the olfactory receptor U200, compared to using data after the molecular structure is identified as output information. In addition, the conversion device U1 can provide a higher coefficient of determination (better accuracy) because it uses data that is closer to post-detection data (raw data) compared to a case where data after the molecular structure is identified is used as the output information.

The above describes preferred embodiments of each of the conversion device, prediction model creation device, conversion information creation method, prediction model creation method, and program of the present invention.

For example, in the fourth embodiment described above, a gas chromatography-mass spectrometer is used as the gas detection device U100, but no limitation is intended. The gas detection device U100 may also be a device that is not equipped with gas chromatography. The gas detection device U100 may also be a direct ionization mass spectrometer (DART-MS). The gas detection device U100 may be an atmospheric mass spectrometer that analyzes at atmospheric pressure.

In the embodiments described above, it is assumed that the conversion unit U12 uses a machine-learned prediction model for conversion, but no limitation is intended. The conversion unit U12 may perform conversion without using a machine-learned prediction model.

### (Other)

The series of processes described above can be executed by hardware or by software, for example. In other words, the functional configuration described above is only an example and no limitation is intended. That is, the information processing system need only be equipped with a function that can execute the series of processes described above as a whole, and the examples above are not limited to the functional blocks that are used to realize this function. The location of the functional blocks is also not limited and can be arbitrary. For example, the functional blocks of the server (information processing device) may be transferred to another device, for example. Conversely, the functional blocks of other devices may be transferred to a server or other device. One functional block may consist of hardware alone, software alone, or a combination of the two.

When the series of processes are executed by software, a program comprising the software is installed on a computer or other device from a network or recording medium. The computer may be a computer embedded in dedicated hardware. The computer may also be a computer capable of performing various functions by installing various programs, as well as a general-purpose smartphone or personal computer for a server.

The recording medium containing such a program consists not only of a removable medium not shown in the figures that is distributed separately from the main unit of the device to provide the program to users and the like, but also of recording media and the like that are provided to users and the like with the program pre-embedded in the main unit of the device. Since the program can be distributed over a network, the recording medium may be mounted on or accessible to a computer connected or capable of being connected to the network.

Herein, the steps describing a program recorded on a recording medium include not only processing that is performed chronologically according to the sequence but also processing that is performed in parallel or individually without necessarily being processed chronologically. Herein, the term "system" means an overall device consisting of multiple devices or multiple means.

In other words, the information processing equipment to which this invention is applied can take various different embodiments having the following configurations. That is, (1) an information processing device including: an acquisition unit configured to acquire receptor response information indicating responses of a plurality of types of olfactory receptors or taste receptors; a storage unit configured to store the receptor response information; an emission information determination unit configured to determine emission information related to emission of a plurality of types of substances related to reception of scent or taste according to the receptor response information; and an emission control unit configured to cause emission of at least one substance of the plurality of types of substances related to reception of scent or taste based on the emission information. This allows the target scent to be reproduced in a non-proprietary manner.

Further, (2) the receptor response information may be information indicating a feature related to scent molecules or taste at a plurality of types of the receptors, the feature being at least one of response intensity, area of response intensity, response durability, response speed, peak time, response rise, and/or number of peaks. This allows for accurate reproduction of the target scent or taste. Since the way flavors or seasonings spread and the temperature at which they are perceived by people can affect the smell or taste, it is better to include more of the above information to accurately reproduce the target scent or taste.

Further, (3) the storage unit may include a correspondence table for associating the receptor response information with the emission information, and the emission information determination unit may determine the emission information based on the receptor response information and the correspondence table. With this configuration, the target scent or taste can be reproduced in a non-attributable manner because the response information can be converted to the corresponding emission information once the response information is determined.

Further, (4) the storage unit may further store a learning model generated in advance by machine learning using the receptor response information and the emission information corresponding to the receptor response information as training data, and the emission information determination unit may use the learning model to determine the emission information based on the receptor response information acquired by the acquisition unit. With this configuration, the target scent or taste can be reproduced without relying on a personal skill by preparing correspondence data (training data), even when it is difficult to generate the correspondence table as described above.

Further, (5) the emission information determination unit may determine at least one type of emission information among emission time, emission amount, emission temperature, emission frequency, and emission port aperture for the substance related to scent or taste reception. With this configuration, the target scent or taste can be reproduced with high accuracy.

Further, (6) the emission information determination unit may determine the emission information according to the receptor response information and spatial parameters related to a state of a space in which the substance is emitted or a positional relationship with an object to which the substance is emitted. With this configuration, for example, when the position of a person's nose (face) can be identified to some extent (direct spraying), such as in a movie theater, the spray direction, spray volume, spray density and the like can be controlled based on the distance to the nose, temperature, humidity and the like. For example, in the case of a large space such as a sports stadium (indirect spraying), the spray direction, spray volume, spray concentration and the like can be controlled based on the size of the space, air volume, air direction, temperature, humidity and the like.

The cartridge to which the present invention is applied can have various different embodiments having the following configurations. That is, (7) a cartridge to be filled with a substance related to reception of the scent or taste emitted by the information processing device described above, the cartridge being filled with a composition that makes only a specific receptor selectively respond.

In addition, the emission device (scent spraying device 3) to which the present invention is applied can have various different embodiments having the following configurations. That is, (8) an emission device including a mounting section for mounting the cartridge described above, and an emission port from which the substance related to reception of the scent or taste is emitted based on emission control performed by the emission control unit. With this configuration, the target scent or taste can be reproduced with the appropriate emission.

In addition, the creation method to which the present invention is applied can have various different embodiments, each having the following structure. That is, (9) a creation method for a scent or taste, the method including a step of emitting a substance related to reception of the scent or taste by the emission device described above.

In addition, the data structure to which the present invention is applied can have various different embodiments, each having the following structure. That is, (10) a data structure containing the receptor response information used in the information processing device described above.

In addition, the generation method to which the present invention is applied can have various different embodiments, each having the following structure. That is, (11) a generation method of generating an N-dimensional code, the method including an encoding step of converting the receptor response information into the N-dimensional code by performing predetermined encoding on the receptor response information described above.

In addition, the generation method to which the present invention is applied can have various different embodiments, each having the following structure. That is, (12) a generation method of generating decoded receptor response information, the method including a decoding step of converting the N-dimensional code into the receptor response information by performing predetermined decoding on the N-dimensional code converted by predetermined encoding on the receptor response information described above.

In addition, the emission device to which the invention is applied can have various different embodiments, each having the following configuration. That is, (13) an emission device including a mounting section for removably mounting a cartridge to be filled with a substance related to reception of scent or taste, an emission control unit configured to, based on emission information related to emission of a plurality of types of substances related to reception of scent or taste, cause emission of at least one substance of the plurality of types of substances related to reception of scent or taste from the cartridge; and an emission port from which the substance related to reception of scent or taste is emitted based on emission control performed by the emission control unit, in which the emission control unit reads information stored in the mounted cartridge and controls a process of the emission upon the cartridge satisfying a predetermined criterion.

The cartridge to which the present invention is applied can have various different embodiments, each having the following configuration. That is, (14) a cartridge configured to be mounted to an emission device including a mounting section for removably mounting the cartridge to be filled with a substance related to reception of scent or taste, an emission control unit configured to, based on emission information related to emission of a plurality of types of substances related to reception of scent or taste, cause emission of at least one substance of the plurality of types of substances related to reception of scent or taste from the cartridge, and an emission port from which the substance related to reception of scent or taste is emitted based on emission control performed by the emission control unit, the emission control unit reading the information stored in the attached cartridge and controlling a process of the emission upon the cartridge satisfying a predetermined criterion, the cartridge including: a case attachable to the mounting section, the case containing the plurality of types of substances related to reception of scent or taste; a non-volatile memory configured to store attribute information corresponding to the predetermined criterion; and an interface for transferring/receiving data indicating the information to/from the emission control unit.

The control method to which the present invention is applied can have various different embodiments, each having the following structure. That is, (15) a control method for an information processing device, the method including: an acquisition step of acquiring receptor response information indicating responses of a plurality of types of olfactory receptors or taste receptors; an emission information determination step of determining emission information related to emission of substances related to reception of a plurality of types of scents or tastes according to the receptor response information; and an emission control step of causing emission of at least one substance of the plurality of types of substances related to reception of scent or taste based on the emission information.

In addition, the computer program to which the present invention is applied can have various different embodiments, each having the following structure. That is, (16) a computer program to cause a computer to execute an acquisition step of acquiring receptor response information indicating responses of a plurality of types of olfactory receptors or taste receptors; an emission information determination step of determining emission information related to emission of substances related to reception of a plurality of types of scents or tastes according to the receptor response information; and an emission control step of causing emission of at least one substance of the plurality of types of substances related to reception of scent or taste based on the emission information.

### EXPLANATION OF REFERENCE NUMERALS

1: Server, 2: Receptor information determination device, 3: Scent spraying device, 11: CPU, 18: Storage unit, 19: Communication unit, 31: Response information acquisition unit, 32: Captured image acquisition unit, 33: Model information acquisition unit, 34: Spray information determination unit, 35: Spray control unit, 36: Display control unit, 41: Response information DB, 42: Model information DB, 43: Correspondence DB, CO: Scent Collector, CA: Cartridge, NO: Nozzle, RE: Content playback device, TV: Display device

## Claims

1. An information processing device comprising:
an acquisition unit that acquires receptor response information indicating responses of a plurality of types of olfactory receptors or taste receptors;
a storage unit that stores the receptor response information;
an emission information determination unit that determines emission information related to emission of a plurality of types of substances related to reception of scent or taste according to the receptor response information; and
an emission control unit that causes emission of at least one substance of the plurality of types of substances related to reception of scent or taste based on the emission information.

2. The information processing device according to claim 1, wherein the receptor response information is information indicating a feature related to scent molecules or taste at a plurality of types of the receptors, the feature being at least one of response intensity, area of response intensity, response durability, response speed, peak time, response rise, and/or number of peaks.

3. The information processing device according to claim 1, wherein:
the storage unit includes a correspondence table for associating the receptor response information with the emission information, and
the emission information determination unit determines the emission information based on the receptor response information and the correspondence table.

4. The information processing device according to claim 1, wherein:
the storage unit further stores a learning model generated in advance by machine learning using the receptor response information and the emission information corresponding to the receptor response information as training data, and
the emission information determination unit uses the learning model to determine the emission information based on the receptor response information acquired by the acquisition unit.

5. The information processing device according to claim 1, wherein the emission information determination unit determines at least one type of emission information among emission time, emission amount, emission temperature, emission frequency, and/or emission port aperture for the substance related to scent or taste reception.

6. The information processing device according to claim 1, wherein the emission information determination unit determines the emission information according to the receptor response information and spatial parameters related to a state of a space in which the substance is emitted or a positional relationship with an object to which the substance is emitted.

7. A cartridge to be filled with a substance related to reception of the scent or taste emitted by the information processing device according to any one of claims 1 to 6,
the cartridge being filled with a composition that makes only a specific receptor selectively respond.

8. An emission device comprising:
a mounting section for mounting the cartridge according to claim 7; and
an emission port from which the substance related to reception of the scent or taste is emitted based on emission control performed by the emission control unit.

9. A creation method for a scent or taste, the method comprising emitting a substance related to reception of the scent or taste by the emission device according to claim 8.

10. A data structure containing the receptor response information used in the information processing device according to any one of claims 1 to 6.

11. A generation method of generating an N-dimensional code, the method comprising converting the receptor response information into the N-dimensional code by performing predetermined encoding on the receptor response information according to claim 10.

12. A generation method of generating decoded receptor response information, the method comprising converting the N-dimensional code into the receptor response information by performing predetermined decoding on the N-dimensional code converted by predetermined encoding on the receptor response information according to claim 10.

13. An emission device comprising:
a mounting section for removably mounting a cartridge to be filled with a substance related to reception of scent or taste,
an emission control unit that, based on emission information related to emission of a plurality of types of substances related to reception of scent or taste, causes emission of at least one substance of the plurality of types of substances related to reception of scent or taste from the cartridge; and
an emission port from which the substance related to reception of scent or taste is emitted based on emission control performed by the emission control unit, wherein
the emission control unit reads information stored in the mounted cartridge and controls a process of the emission upon the cartridge satisfying a predetermined criterion.

14. A cartridge to be mounted to an emission device comprising a mounting section for removably mounting the cartridge to be filled with a substance related to reception of scent or taste, an emission control unit that, based on emission information related to emission of a plurality of types of substances related to reception of scent or taste, causes emission of at least one substance of the plurality of types of substances related to reception of scent or taste from the cartridge, and an emission port from which the substance related to reception of scent or taste is emitted based on emission control performed by the emission control unit, the emission control unit reading the information stored in the attached cartridge and controlling a process of the emission upon the cartridge satisfying a predetermined criterion, the cartridge comprising:
a case attachable to the mounting section, the case containing the plurality of types of substances related to reception of scent or taste;
a non-volatile memory that stores attribute information corresponding to the predetermined criterion; and
an interface for transferring/receiving data indicating the information to/from the emission control unit.

15. A control method for an information processing device, the method comprising: acquiring receptor response information indicating responses of a plurality of types of olfactory receptors or taste receptors;
determining emission information related to emission of substances related to reception of a plurality of types of scents or tastes according to the receptor response information; and
causing emission of at least one substance of the plurality of types of substances related to reception of scent or taste based on the emission information.

16. A computer program that causes a computer to execute:
acquiring receptor response information indicating responses of a plurality of types of olfactory receptors or taste receptors;
determining emission information related to emission of substances related to reception of a plurality of types of scents or tastes according to the receptor response information; and
causing emission of at least one substance of the plurality of types of substances related to reception of scent or taste based on the emission information.
